# EUROPEAN PATENT APPLICATION

(11) **EP 4 779 035 A2**
(43) Date of publication of application: **22.07.2026**
(21) Application number: 26170048.8
(22) Date of filing: 24.01.2024
(51) Int. Cl.: C12Q 1/6886

(54) **METHOD FOR THE TARGETED INTEGRATION OF UNIQUE MOLECULAR IDENTIFIERS (UMIS) AND THE HIGH PRECISION DETECTION OF MEDICAL CONDITIONS**

(30) Priority: 30.01.2023 BE 202305056
(62) Divisional of application: 24702684.2
(71) Applicant: Grand Hopital de Charleroi, 6060 Charleroi (BE)
(72) Inventor: CARRASCO, Javier, 6000 Charleroi (BE)
(74) Representative: Willnegger, Eva

(57) **Abstract**

A high precision method and system for the detection of a medical or cosmetic condition. A unique molecular identifiers (UMI) is attached to a targeted DNA sequence comprising a region of interest (ROI) in a targeted manner. A NGS library enrichment using 2 nested ROI-specific enrichment PCR amplifications is performed followed by NGS sequencing. The precision to detect and characterize T cell receptors (TCR) diversity is substantially increased. This is particularly relevant for TCR monitoring applications such as cancer.

## Description

### FIELD OF THE INVENTION

The present invention concerns the field of high precision detection of medical or cosmetic conditions, in particular the detection of cancer.

### BACKGROUND

Cancer is a complex multigenic disease. Diverse genetic alterations characterize this condition. High precision diagnostics help detect the onset, progression or recidivity of cancer. This facilitates a more personalized approach to clinical care through risk stratification and treatment selection.

Next-generation sequencing (NGS) allows for the determination of unique gene expression signatures for each tumor. This signature contributes to both disease classification, diagnostics, and prognostics.

For example, US2022195493A1 to Cellecta describes the multiplex targeted PCR through the preparation of barcoded gene specific DNA fragments. The method includes employing a set of gene specific primer pairs, wherein each pair of gene specific primers is made up of a forward primer and a reverse primer, at least one of which includes a sample barcode domain. The sample barcoded nucleic acid has the following structure: 3-linker 1-sample barcode domain-anchor 2 domain-RNA binding domain-5. However, this document does not describe a method comprising the integration of UMI integration primer, followed by two consecutive nested enrichment PCR amplifications.

US2021355533A1 to Tempus Labs discloses profiling T cell receptor (TCR) and B cell receptor (BCR) repertoire using next-generation sequencing (NGS) methods and hybrid-capture probes. The hybrid capture probes comprise, in order from 5' to 3', an amplification region, a sample tag region, a UMI, and an anchor region. Therefore, there is still a need for further improved cancer diagnostics.

### SHORT DESCRIPTION OF THE INVENTION

The inventors have surprisingly found that the precision to detect and characterize T cell receptors (TCR) diversity is substantially increased by
▪ integrating the UMI upstream or downstream of a region of interest (ROI) and
▪ performing a NGS library enrichment using 2 nested ROI specific PCR amplifications.

This is particularly relevant for TCR monitoring applications such as cancer.

Accordingly, a first aspect of the invention is A high precision method for the detection of a medical or cosmetic condition, comprising:
A. Providing a patient sample comprising a targeted DNA sequence (10); wherein the targeted DNA sequence is from 20 to 200 bases upstream and from 20 to 200 base pairs downstream from the center of a region of interest (ROI, 20), wherein the ROI (20) consists of 1 to 100, preferably from 10 to 50, and even more preferably from 20 to 50 bases; and wherein the ROI (20) is associated with the medical or cosmetic condition; and attaching a unique molecular identifier (UMI, 33, 33') to the targeted DNA sequence (10) using a UMI integration primer (30, 30'); wherein the UMI integration primer (30, 30') comprises:
   ▪ an amplification adapter (31, 31'),
   ▪ a Unique Molecular Identifier (UMI, 33, 33'),
   ▪ a sample identifier (32, 32') ; and
   ▪ a DNA sequence specific for the targeted DNA sequence (34, 34'), wherein 3'-end is neighboring the targeted DNA sequence (10).
B. Amplifying the targeted DNA sequence (10) through a first enrichment PCR thereby obtaining a first enrichment amplicon of 200 to 700 base pairs, wherein the first enrichment amplicon comprises the ROI (20);
C. Amplifying the targeted DNA sequence (10) through a second nested enrichment PCR thereby obtaining a second enrichment amplicon of 200 to 500 base pairs, wherein the second enrichment amplicon comprises the ROI (20);
D. Attaching NGS adapters (60, 61) to the second enrichment PCR amplicons; and
E. Sequencing the second enrichment amplicons through Next Generation Sequencing (NGS);
   wherein the first enrichment PCR comprises amplification with:
      ▪ one or more first enrichment PCR primers (40), wherein the first enrichment PCR primers (40) are specific for the targeted DNA sequence (10); wherein the first enrichment PCR primers (40) are forward if the UMI integration primer (30) is downstream from the ROI (20); and wherein the first enrichment PCR primers are reverse if the UMI integration primer (30') is upstream from the ROI (20); and
      ▪ a primer (41) complementary to the amplification adapter (31);
   wherein the second enrichment PCR comprises amplification with:
      ▪ one or more second enrichment PCR primers (50), wherein the second enrichment PCR primers (50) are specific for the ROI (20) or for the targeted DNA sequences (10), wherein the second enrichment PCR primers (50) are nested with respect to first enrichment PCR primers (40); and wherein the second enrichment PCR primer (50) comprises a generic adapter (51) at the 5'-end; and
      ▪ a primer (52) complementary to the amplification adapter (31), wherein the primer (52) comprises a generic adapter (53).

In another aspect, the targeted DNA sequence (10) has a length of 20 to 200 nucleotides, preferably 20 to 100 nucleotides from the center of the ROI.

In another aspect, the ROI (20) consists of 1 to 100, preferably from 10 to 50, and even more preferably from 20 to 50 bases.

In another aspect, the DNA sequence specific for the targeted DNA sequence (34, 34') has a length of 10 to 60 nucleotides, preferably 15 to 25 nucleotides, and even more preferably 18 to 24 nucleotides.

In another aspect, the UMI has a length of 5 to 20 nucleotides, preferably 8 to 18 nucleotides, even more preferably 12 to 15, and even more preferably 14 nucleotides.

In another aspect, the UMI is an aleatory sequence, a semi-aleatory sequence or a combination thereof.

In another aspect, the random portion of the semi-aleatory sequence is from 2 to 12 bases.

In another aspect, the UMI consists of the following consecutive base order:
▪ 4 random bases,
▪ one fixed base,
▪ 4 random bases,
▪ one fixed base, and
▪ 4 random bases.

In another aspect, the region of interest is a T-cell receptor or a B-cell receptor, preferably a T-cell receptor.

In another aspect, the medical condition is cancer, preferably breast cancer or gastrointestinal cancer.

In another aspect, the medical condition is recidivist cancer.

In another aspect, the method comprises the determination of a treatment in function of the detected region of interest.

Another aspect is a system for the high precision detection of a medical or cosmetic condition, comprising means configured to perform the following steps:
A. Providing a patient sample comprising a targeted DNA sequence (10); wherein the targeted DNA sequence is from 20 to 200 bases upstream and from 20 to 200 base pairs downstream from the center of a region of interest (ROI, 20), wherein the ROI (20) consists of 1 to 100, preferably from 10 to 50, and even more preferably from 20 to 50 bases; and wherein the ROI (20) is associated with the medical or cosmetic condition; and attaching a unique molecular identifier (UMI, 33, 33') to the targeted DNA sequence (10) using a UMI integration primer (30, 30'); wherein the UMI integration primer (30, 30') comprises:
   ▪ an amplification adapter (31, 31'),
   ▪ a Unique Molecular Identifier (UMI, 33, 33'),
   ▪ a sample identifier (32, 32') ; and
   ▪ a DNA sequence specific for the targeted DNA sequence (34, 34'), wherein 3'-end is neighboring the targeted DNA sequence (10).
B. Amplifying the targeted DNA sequence (10) through a first enrichment PCR thereby obtaining a first enrichment amplicon of 200 to 700 base pairs, wherein the first enrichment amplicon comprises the ROI (20);
C. Amplifying the targeted DNA sequence (10) through a second nested enrichment PCR thereby obtaining a second enrichment amplicon of 200 to 500 base pairs, wherein the second enrichment amplicon comprises the ROI (20);
D. Attaching NGS adapters (60, 61) to the second enrichment PCR amplicons; and
E. Sequencing the second enrichment amplicons through Next Generation Sequencing (NGS);
   wherein the first enrichment PCR comprises amplification with:
      ▪ one or more first enrichment PCR primers (40), wherein the first enrichment PCR primers (40) are specific for the targeted DNA sequence (10); wherein the first enrichment PCR primers (40) are forward if the UMI integration primer (30) is downstream from the ROI (20); and wherein the first enrichment PCR primers are reverse if the UMI integration primer (30') is upstream from the ROI (20); and
      ▪ a primer (41) complementary to the amplification adapter (31);
   wherein the second enrichment PCR comprises amplification with:
      ▪ one or more second enrichment PCR primers (50), wherein the second enrichment PCR primers (50) are specific for the ROI (20) or for the targeted DNA sequences (10), wherein the second enrichment PCR primers (50) are nested with respect to first enrichment PCR primers (40); and wherein the second enrichment PCR primer (50) comprises a generic adapter (51) at the 5'-end; and
      ▪ a primer (52) complementary to the amplification adapter (31), wherein the primer (52) comprises a generic adapter (53).

### DETAILED DESCRIPTION OF THE INVENTION

The invention will now be described in further details:

### TCR

"TCR" or "t-cell receptor" refers to a protein complex found on the surface of T cells, or T lymphocytes, that is responsible for recognizing fragments of antigen as peptides bound to major histocompatibility complex (MEW) molecules. In some embodiments, the term TCR or T-cell receptor refers to at least a part of the region of a genome responsible for the development of T-cell receptor.

The diversity of TCRs is achieved by the process of recombination. Upon antigen presentation by innate immune cells, lymphocytes that bear receptors with high affinity to antigen are activated and clonally expand. Activated lymphocytes differentiate to become effector cells. After an infection, memory cells may persist for the lifetime of the host.

The variety of lymphocyte receptors of a subject are generally referred to as the "repertoire." Therefore, the repertoire of lymphocyte receptors represents a fingerprint of a response to diseases such as cancer. This fingerprinting provides a record of past immunological challenges. For blood cancers, the repertoire can be monitored for the "tumor" or cancer cells. For solid cancers, the repertoire can be used to study why the immune system is not recognizing/eliminating the cancer cells as non-self cells. For at least these reasons, there is great interest in the profile of an individual subject's lymphocyte receptors.

### BCR

"BCR" or "B-cell receptor" refers to immunoglobulin molecules that form a receptor protein usually located on the outer surface of a lymphocyte type known as a "B-cell". In some embodiments, the term BCR or B-cell receptor refers to at least a part of the region of a genome responsible for the development of B-cell receptor.

### TCR/BCR repertoire

"TCR/BCR repertoire" refers to the totality of information derived from nucleic acid sequencing of a sample isolated from a subject/patient using the method of the present invention. A TCR/BCR repertoire includes information, for example in the form of sequence data, about target genes. Such information can be analyzed by methods known in the art to determine receptor types.

### V(D)J

"V(D)J recombination" refers to the nearly random rearrangement of variable (V), joining (J), and diversity (D) gene segments. This results in a variety of amino acid sequences in the antigen-binding regions of immunoglobulins and TCRs that allow for the recognition of antigens from pathogens as well as cancer cells.

"V region" refers to a BCR or TCR variable gene segment or gene product thereof.

"D region" refers to a BCR or TCR diversity gene segment or gene product thereof.

"J region" refers to a BCR or TCR joining gene segment or gene product thereof.

"C region" refers to a BCR or TCR constant gene segment or gene product thereof.

### Next Generation Sequencing (NGS)

Immune profiling leverages Next Generation Sequencing (NGS) to accurately sequence the immune receptor repertoire. NGS can produce sequencing "reads". These reads are then aligned to a reference genome or transcriptome to give a relatively complete picture of a genome or a sample's transcriptome.

NGS usually has an error rate of roughly 0.5-0.2%. That means that from 1/200 to 1/500 bases are incorrect. Molecular annotation strategies are used to detect variants and other mutations that occur at lower frequencies.

Assembling and analyzing reads of T- and B-cell receptors remains challenging. High quality sequencing data depends on the breadth and depth of sequencing. The breadth refers to the number of genome bases that are covered by the sequencing. The depth refers to how many times a particular base or region is covered by the sequencing run.

However, the presence of transcripts encoding lymphocyte receptors can be very limited. Therefore, obtaining deep sequencing results that accurately represent the individual subject's T- and B-cell repertoire, without directly enriching or selecting for the non-constant regions of TCRs and BCRs, can be challenging.

"Read" refers to a DNA sequence of at least 30 bases. Reads can be used to identify a larger sequence or region by aligning it with a chromosome, genomic region, or gene. A read may be a paired-end or single-end read.

"Aligned", "alignment", or "aligning" refer to a process used to identify regions of similarity. Alignment refers to matching sequences with positions in a reference genome based on the order of their nucleotides in these sequences. Alignment can be performed manually or by a computer algorithm. A preferred algorithm is the Efficient Local Alignment of Nucleotide Data (ELAND) app distributed by Illumina. Alignment can refer to either a 100% sequence match or to a match that is less than 100% (non-perfect match). Alignment can include pseudo-alignment.

### Library

"Library" and "sequencing library" refer to a pool of DNA fragments with adapters attached.

### NGS Adapter

"NGS Adapters" are commonly designed to interact with a specific sequencing platform, e.g., the surface of a flow-cell (Illumina) or beads (Ion Torrent), to facilitate a sequencing reaction.

### Variant

"Variant" means a difference in a genetic sequence or genetic profile, as compared to a reference genome or reference genetic profile.

### Expression level

"Expression level" is used herein to describe the number of copies of a particular RNA or protein molecule generated by a gene or other genetic regulatory region. The expression level may be normalized using standard methods. Such methods include counts per million or finding the base 10 logarithm of the raw read count. This may be defined by a chromosomal location or other genetic mapping indicator.

### Targeted DNA sequence and region of interest (ROI)

In one embodiment, the "Targeted DNA sequence" can be any partial or full gene relevant in the development, detection or prevention of medical or cosmetic conditions, diseases or disorders. In one embodiment, the target gene is a cancer gene or a gene involved in immunology.

The "targeted DNA sequence" is characteristic of the region of interest (ROI), preferably of a conserved region of the ROI.

In a preferred embodiment, the targeted DNA sequence has a length of 20 to 200 bases from the center of the ROI, preferably 20 to 100 bases from the center of the ROI.

The targeted DNA sequence comprises the "region of interest."

The "region of interest" usually is a variant of the target gene or a part of the target gene. The terms "region of interest" and "targeted region" are used interchangeably.

In one embodiment, the ROI (20) consists of 1 to 100, preferably from 10 to 50, and even more preferably from 20 to 50 bases.

In another embodiment, the ROI (20) consists only of 1 to 5 bases, such as for example 1 base.

In one embodiment, the ROI (20) is associated with the medical or cosmetic condition;

### Distance from ROI

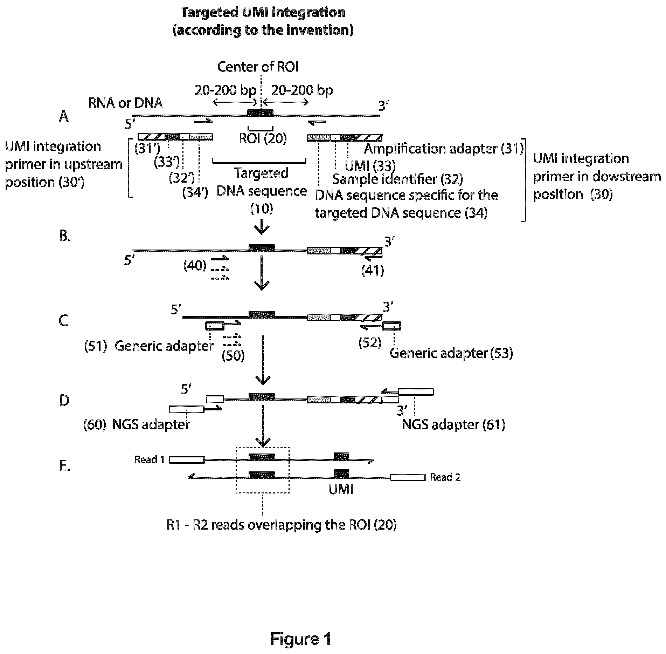

The distance from the ROI is calculated as the distance from the center of the ROI as shown in Figure 1. If the ROI is a single nucleotide, the distance from the ROI is calculated from this nucleotide. In case of an even base number of the ROI, the distance is calculated from half of the length of the ROI. For a ROI of 20 bases, the distance at base 10 in reading direction. For an ROI of 21 bases, the distance is calculated from base 10 in reading direction. For an ROI of 22 bases, the distance is calculated from base 11 in reading direction. For an ROI of 23 bases, the distance is calculated from base 12 in reading direction.

### DNA sequence specific for the targeted DNA sequence

In one embodiment, the DNA sequence specific for the targeted DNA sequence has a length of 10 to 60 nucleotides, preferably 15 to 25 nucleotides, and even more preferably 18 to 24 nucleotides.

### Bases and nucleotides

The terms "bases" and "nucleotides" are used interchangeably in the context of the present invention.

### Primer

A "primer" is a short single-stranded nucleic acid that initiates DNA synthesis and replication through a DNA polymerase.

The primer of the present invention are preferably chemically synthesized oligonucleotides, preferably DNA oligonucleotides. A typical length of the primer is 10 to 30 nucleotides, preferably 15 to 25 nucleotides, preferably between 18 and 24 nucleotides.

### Upstream

"Upstream" is in general toward the 5'-end of the coding strand for the targeted DNA sequence.

### Downstream

"Downstream" is toward the 3'-end of the coding strand for the targeted DNA sequence.

### Targeted UMI integration

The "UMI integration" according to the present invention is targeted. The targeted integration of the UMI is achieved through a UMI integration primer (30, 30'). The UMI integration primer (30, 30') is designed in a way to allow for the integration of the UMI upstream or downstream in distance ranging from 20 to 200 base pairs from the center of the ROI (20). The UMI integration primer (30, 30') typically comprises and preferably consists of:
▪ an amplification adapter (31, 31'),
▪ a Unique Molecular Identifier (UMI, 33, 33'),
▪ a sample identifier (32, 32') ; and
▪ a DNA sequence specific for the targeted DNA sequence (34, 34'), wherein 3'-end is neighboring the targeted DNA sequence (10).

In one embodiment, the UMI has a length of 5 to 20 nucleotides, preferably 8 to 18 nucleotides, even more preferably 12 to 15, and even more preferably 14 nucleotides.

In one embodiment, the UMI is an aleatory sequence, a semi-aleatory sequence or a combination thereof.

In one embodiment, the random portion of the semi-aleatory sequence is from 2 to 12 bases.

In one embodiment, the UMI consists of the following consecutive base order:
▪ 4 random bases,
▪ one fixed base,
▪ 4 random bases,
▪ one fixed base, and
▪ 4 random bases.

In one embodiment, the primer is designed to match a conserved region usually upstream of the targeted region of the target gene or the region of interest.

In another embodiment, the UMI integration primer is a degenerate primer.

In a preferred embodiment, the UMI is integrated at the 3'-end of the DNA or RNA sequence.

Starting from RNA, the UMI is usually integrated through a reverse transcriptase.

### PCR enrichment

PCR can be performed using any standard PCR reaction conditions.

"Enrichment" preferably is sequence specific through target-specific PCR primers. However, nonspecific enrichment involving any of the nucleic acids present in a sample may also be included.

"Enriched" refers to the level or an amount of one or more biomolecules in a sample. "Enriched" may also refer to an increased level or amount of the one or more biomolecules as compared to a control level. "Enriched" may also refer the relative abundance as compared to the other biomolecules in the sample (relative amount). In a data science context, "enrichment" refers to statistical enrichment.

In a preferred embodiment two or more PCR enrichments are carried out:
▪ a first PCR enrichment; and
▪ a second nested PCR enrichment.

### First enrichment PCR

The first enrichment PCR comprises amplifying the targeted DNA sequence (10) to obtain a first enrichment amplicon of 200 to 700 base pairs, wherein the first enrichment amplicon comprises the ROI (20). The first enrichment PCR comprises amplification with:
▪ one or more first enrichment PCR primers (40), wherein the first enrichment PCR primers (40) are specific for the targeted DNA sequence (10); wherein the first enrichment PCR primers (40) are forward if the UMI integration primer (30) is downstream from the ROI (20); and wherein the first enrichment PCR primers are reverse if the UMI integration primer (30') is upstream from the ROI (20); and
▪ a primer (41) complementary to the amplification adapter (31);

### Second nested enrichment PCR

The second enrichment PCR comprises amplifying the targeted DNA sequence (10) to obtain a second enrichment amplicon of 200 to 500 base pairs, wherein the second enrichment amplicon comprises the ROI (20).

The second enrichment PCR comprises amplification with:
▪ one or more second enrichment PCR primers (50), wherein the second enrichment PCR primers (50) are specific for the ROI (20) or for the targeted DNA sequence (10), wherein the second enrichment PCR primers (50) are nested with respect to first enrichment PCR primers (40); and wherein the second enrichment PCR primer (50) comprises a generic adapter (51) at the 5'-end; and
▪ a primer (52) complementary to the amplification adapter (31), wherein the primer (52) comprises a generic adapter (53).

Through this second nested PCR amplification, the precision can by substantially increased.

### Use for detecting a medical or cosmetic condition

The method of the present invention is useful for detecting medical or a cosmetic condition. The method of the present invention is useful as a biomarker.

### Biomarker

In one embodiment the method of the present invention is used as biomarker or as a companion diagnostic.

"Biomarker" means any genetic variant or molecule or set of molecules, or characteristic of a molecule. It may include location or expression level. The biomarker is indicative of or correlated with a condition of interest as follows:
▪ the existence of an infection,
▪ a medical condition or disease, such as cancer,
▪ the susceptibility to an infection, conditions, or disease in the subject,
▪ the likelihood that the infection, medical condition, or disease is one subtype vs. another,
▪ the probability that a patient will or will not respond to a particular therapy or class of therapy,
▪ the degree of the positive response that would be expected for a therapy or class of therapies (e.g., survival and/or progression-free survival, which may be quantified as an interval of time),
▪ whether a patient is responding to a therapy, or the likelihood that an infection, medical condition, or disease has progressed or will progress, or has or will progress beyond its site of origin (i.e., metastasize),

In a preferred embodiment, the method of the present invention is used to determine the recidivist risk of a cancer patient.

The biomarker preferably comprises a TCR/BCR repertoire or profile.

The medical or cosmetic condition can be any condition. Preferably, the medical condition is cancer.

### Cancer

"Cancer" refers to benign or malignant tumors. Tumors may be capable of invasive growth and metastases through a human or animal body or a part thereof. Tumors may form metastases via the lymphatic system and/or the blood stream. Tumors also include solid tumors. Typical cancers include carcinomas, lymphomas, or sarcomas. Examples are skin cancer, ovarian cancer, colon cancer, breast cancer, pancreatic cancer, lung cancer, prostate cancer, urinary tract cancer, uterine cancer, acute lymphatic leukemia, Hodgkin's disease, small cell carcinoma of the lung, melanoma, neuroblastoma, glioma, and soft tissue sarcoma of humans.

Exemplary cancers that can be detected, identified, predicted, diagnosed, or monitored with method of the present invention include skin cancer, B-cell cancer, e.g., multiple myeloma, melanomas, breast cancer, lung cancer (such as non-small cell lung carcinoma or NSCLC), bronchus cancer, colorectal cancer, prostate cancer, pancreatic cancer, stomach cancer, ovarian cancer, urinary bladder cancer, brain or central nervous system cancer, peripheral nervous system cancer, esophageal cancer, cervical cancer, uterine or endometrial cancer, cancer of the oral cavity or pharynx, liver cancer, kidney cancer, testicular cancer, biliary tract cancer, small bowel or appendix cancer, salivary gland cancer, thyroid gland cancer, adrenal gland cancer, osteosarcoma, chondrosarcoma, cancer of hematological tissues, adenocarcinomas, inflammatory myofibroblastic tumors, gastrointestinal stromal tumor (GIST), colon cancer, multiple myeloma (MM), myelodysplastic syndrome (MDS), myeloproliferative disorder (MPD), acute lymphocytic leukemia (ALL), acute myelocytic leukemia (AML), chronic myelocytic leukemia (CML), chronic lymphocytic leukemia (CLL), polycythemia Vera, Hodgkin lymphoma, non-Hodgkin lymphoma (NHL), soft-tissue sarcoma, fibrosarcoma, myosarcoma, liposarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, squamous cell carcinoma, basal cell carcinoma, adenocarcinoma, sweat gland carcinoma, sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, seminoma, embryonal carcinoma, Wilms' tumor, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, neuroblastoma, retinoblastoma, follicular lymphoma, diffuse large B-cell lymphoma, mantle cell lymphoma, hepatocellular carcinoma, thyroid cancer, gastric cancer, head and neck cancer, small cell cancers, essential thrombocythemia, agnogenic myeloid metaplasia, hypereosinophilic syndrome, systemic mastocytosis, familiar hypereosinophilia, chronic eosinophilic leukemia, neuroendocrine cancers, or carcinoid tumors.

### Treatment

In one embodiment, the method of the present invention further comprises the determination of a treatment or of a treatment recommendation in function of the detected region of interest.

"Treatment" or "treating" means obtaining a desired pharmacologic and/or physiologic effect. The effect may be prophylactic. Accordingly, in one embodiment, a disease or symptom thereof are completely or partially prevented. In another embodiment, treatment is the partial or complete cure for a disease and/or adverse effect attributable to the disease. "Treatment" as used herein covers any treatment of a disease in a mammal, and includes:
▪ preventing the disease from occurring in a subject which may be susceptible to the disease but has not yet been diagnosed as having it;
▪ inhibiting the disease or the reoccurrence of the disease, i.e., arresting its development; or (c) relieving the disease, i.e., causing regression of the disease. The therapeutic agent may be administered before, during or after the onset of disease or injury.
▪ the treatment of ongoing disease, where the treatment stabilizes or reduces the undesirable clinical symptoms of the patient, is of particular interest. The subject therapy will desirably be administered during the symptomatic stage of the disease, and in some cases after the symptomatic stage of the disease.

### Advantages

The methods and systems of the present invention allow for the high precision quantitative or qualitative detection of mutations, gene deletions, and polymorphisms and the production or quantitation of amplicons for high throughput sequencing. The high precision methods and systems of the present invention allow for the identification of medical or cosmetic conditions in advance of their radiological detectability. Therefore, the method of the present invention is particularly useful in respect of detecting the onset of cancer or of cancer recidivism.

Each T-cell and B-cell is unique at the DNA level. They differ at the T-cell receptor (TCR) gene or B-cell receptor (BCR) gene that determines the antigen that will be recognized by the cell. Assembling and determining the sequences of TCR and BCR genes can help to predict immune responses; diagnose or confirm diseases, conditions, or pathogen exposure; determine disease severity; measure or confirm therapeutic effect and efficacy; determine minimal residual disease (MRD); and provide the information necessary to produce specific therapies or treatments.

### SHORT DESCRIPTION OF THE DRAWINGS

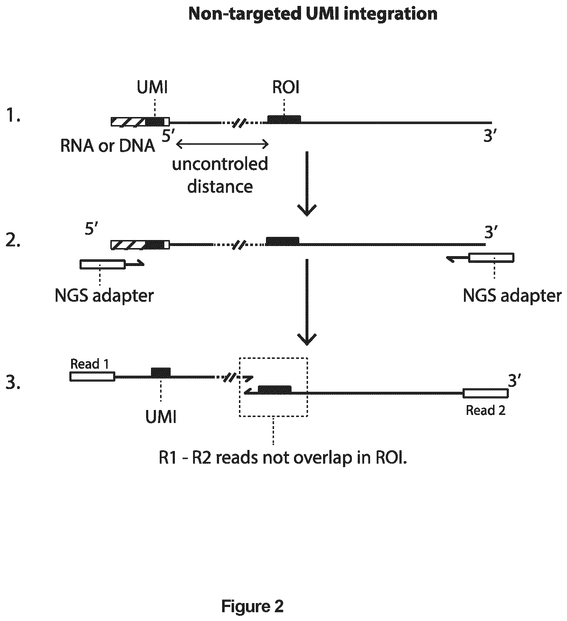

**Figure 1**
   Figure 1 shows a schematic representation of the process of the present invention. In a first step (A), the UMI is integrated using the UMI integration primer containing from 5' to 3', an amplification adapter (31, 31'), a UMI (33, 33'), a sample identifier (32, 32') and a DNA sequence specific for the targeted DNA sequence (34, 34'). When the starting template is RNA, the UMI integration primer is positioned downstream (30) of the target region at a distance ranging from 20-200 base pairs and used in a reverse transcription reaction. When the starting template is a double-stranded DNA, the UMI integration primer can be downstream (30) of the target region at a distance of 20-200 base pairs but also upstream (30') in the same distance interval. In a second step (B), a first PCR amplification is performed using a primer (41) complementary to the amplification adapter (31) of the UMI integration primer (30) and one or more amplification primers (40) specific to the targeted gene(s). These specific primers can be in the forward or reverse position depending on the position of the UMI integration primer in the first step. In a third step (C), a second nested PCR amplification is performed using a primer (52) complementary to the amplification adapter (31) of the UMI integration primer (30) and one or more amplification primers (50) specific to the targeted gene(s). These primers contain a generic adapter at their 5'-end (51, 53). The amplicons obtained after this second nested PCR have a size between 200 and 500 base pairs. The fourth step (D) allows the integration of the NGS adapters (60 and 61) by using primers complementary to the generic adapters (51, 53) introduced during the third step. The obtained amplicons are then sequenced (E) by paired-end NGS to obtain overlapping pair reads in the ROI. This process can be used for the detection of a medical or cosmetic condition. In another embodiment, the method of the present invention can be used as a biomarker.
**Figure 2**
   For comparison, Figure 2 illustrates a method for non-targeted integration of the UMI at the 5' end of a gene fragment, e.g., using a Terminal Switching Oligonucleotide with an UMI during a reverse transcription reaction, according to the prior art. This approach does not allow control of the distance between the UMI position and a ROI, and the size of the amplicons generated during library construction cannot be controlled and their sequencing by pairwise NGS does not guarantee overlapping read pairs in the ROI. The sequence quality is then diminished.
**Figure 3**

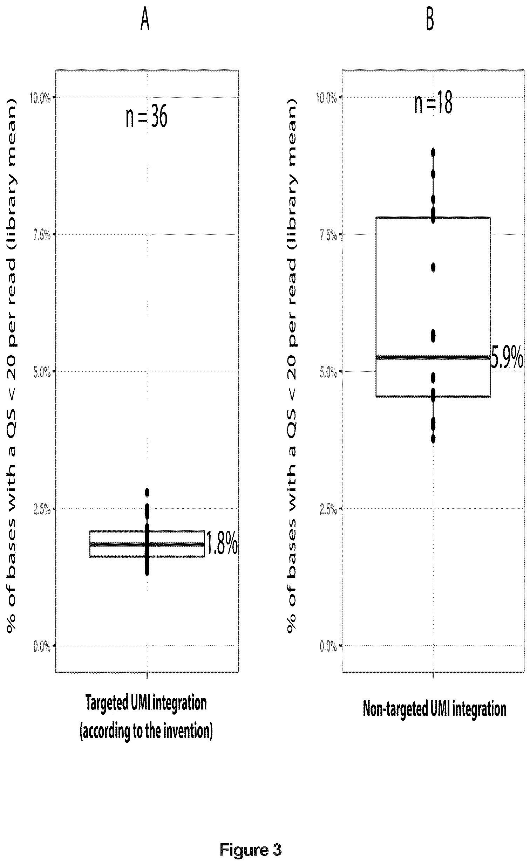

   Figure 3 illustrates the advantage of targeted integration of UMIs using the method described in the invention (Figure 1) over non-targeted integration as described in the prior art (Figure 2) in terms of the quality of sequences obtained by targeted NGS. The targeted genes in the illustrative libraries correspond to the beta TCRs and the ROls correspond to the CDR3 hypervariable region. Very high sequence quality is particularly important for the characterization of these extremely diverse genes. For each library, the quality of the obtained sequences is evaluated by measuring the average percentage of bases with a Phred QS < 20 (implicating in risk of incorrect base call of > 1/ 100) per pair-reads aligned on a beta TCR. The results obtained for 36 libraries in which the UMIs were integrated in a targeted manner in accordance with the described invention are shown in boxplot 3A and those obtained for 18 libraries in which the UMIs were integrated in a non-targeted manner in accordance with the prior art, are shown in boxplot 3B.
**Figure 4**

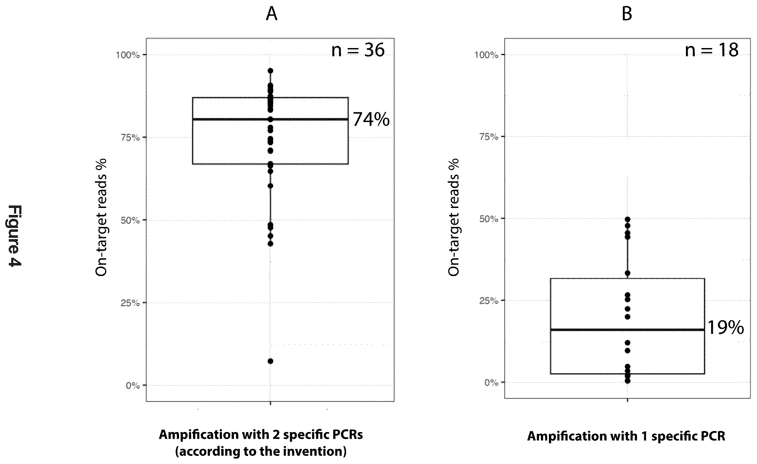

   Figure 4 illustrates the advantage of targeted DNA sequence(s) enrichment during NGS library construction based on two nested PCRs specific for this(those) gene(s) in accordance with the method described in the invention (Figure 1) compared to enrichment based on a single PCR specific for this(those) gene(s). The targeted genes in the illustrative libraries correspond to the TCR beta genes and the ROIs correspond to the CDR3 hypervariable region. High enrichment efficiency is particularly important for the detection of rare TCRs beta rearrangements or the detection of TCRs beta in samples containing few T cells. Enrichment efficiency is evaluated by measuring the percentage of paired reads aligned to TCR beta genes in relation to the total number of raw reads obtained (% of on-target reads). The results obtained for 36 libraries where the enrichment used 2 nested PCRs specific to TCR beta genes in accordance with the described invention are shown in boxplot 4A and those obtained for 18 libraries where the enrichment used 1 single TCR beta specific PCR in accordance with the previous art are shown in boxplot 4B.

### EXAMPLES

### Example 1

Example 1 aims at illustrating the advantage of a targeted integration of UMIs compared to a non-targeted integration in terms of the quality of the NGS sequencing.

### A. A series of NGS libraries targeted to TCR beta genes were constructed UMI integration

For Figure 3A, RNA from a PBMC sample was extracted and engaged in a reverse transcription reaction using an UMI integration primer. The UMI integration primer included from 5' to 3'-end:
▪ 28 nucleotides corresponding to an adapter;
▪ 14 nucleotides including 12 random ones corresponding to the UMI;
▪ 6 nucleotides corresponding to a sample identifier; and
▪ 20 nucleotides corresponding to a specific sequence of the *TRBC1* and *TRBC2* genes (5'

### UMI integration primer sequence:

5’AAGCAGTGGTATCAACGCAGAGTGCAGNNNNUNNNUNNNTAGCTGGTGTGGC
CTTTTGGGTG '3.

The resulting cDNA was purified using magnetic purification beads.

Then, 12 decreasing dilutions were performed. Each was committed to 4 replicates (n = 36) for the construction of an NGS library targeted to TCR beta genes.

### B. First enrichment PCR

A first step of enrichment of these genes was performed by multiplex PCR with forward primers specific to functional *TRB V* genes based on the IMGT data bank (www. IMGT.org). These primers were positioned at 110 to 250 nucleotides from the hypervariable region of the TCR beta (CDR3 region).

For example, the specific sequence for *TRBV2*01* was: 5'ATACTTCTATTGGTACAGACAAATCT.

The reverse primer was complementary to the adapter included at the 5' end of the UMI integration primer:
5'AAGCAGTGGTATCAACGCAGAGT3'.

The products obtained after this first enrichment were purified using magnetic purification beads.

### C. Second nested enrichment PCR

The totality of the amplicons obtained was engaged in a second enrichment PCR. The primers of this second PCR were nested with respect to the primers of the first enrichment PCR.

The forward primers were specific to the targeted *TRB V* gene sequences and are located at a distance of 40 to 70 nucleotides from the ROI.

For example, the specific sequence for *TRBV2*01* was:
5'AAGACTCGGCAGCATCTCCAACTCTGAAGATCCGGTCCACA3'

The forward primer included an adaptor of 21 nucleotides at the 5' position of the primer.

The reverse primer was complementary to the adaptor included at the 5' end of the UMI integration primer:
5'GCGATCGTCACTGTTCTCCAGCAGTGGTATCAACGCAGAGTGC3'

The reverse primer included a 21-nucleotide adaptor in the 5' position. The products of this 2^{nd} PCR were purified with magnetic purification beads.

### D. Attachment of NGS adapters

A fraction of the amplicons obtained was engaged in a simplex PCR. The forward and reverse primers were specific to the adapters included at the 5' ends of the primers used in the second nested enrichment PCR.

We integrated the P5 and P7 adapters as well as the MIDs necessary for sequencing in the MiSeq-Illumina platform.

### E. NGS sequencing of the second enrichment amplicons

The sequencing run was performed following the standard procedure. Illumina's recommended reagents were used in paired-end condition with a run of 2x 250 cycles. The generated reads were read and aligned to the TCR beta genes. The percentage of bases with a Phred QS < 20 is recorded for each aligned read. The probability of incorrect base call was 1/ 100. An average value is calculated for each library. The distribution of the results obtained for the 36 evaluated libraries is represented by a box-plot in Figure 3A.

### Comparative example 1B - Figure 3B: non-targeted UMI integration

For Figure 3B, RNA from a PBMC sample was extracted and engaged in a reverse transcription reaction using a primer specific for the *TRBC1* and *TRBC2* genes:
5'GTGTTCCCACCCGAGGTC3'.

A template Switch Oligo (TSO) is also added during the reverse transcription reaction. It was biotinylated at the 5'-end and had the following design in consecutive order:
▪ an adaptor of 21 bases at the 5'-end,
▪ 12 nucleotides including 10 random ones corresponding to the UMI; and
▪ 3 Guanines at 3'
5'BiosG/TAGCAGAGGATATCAACGCAAGTANNNUNNNUNNNNrGrGrG3' .

These 3 guanines allow the TSO to hybridize to unprocessed C nucleotides added by the reverse transcriptase during reverse transcription. Its sequence is integrated at the 5' end of the cDNA. The distance between the position of the TSO including the UMI and the ROI cannot be controlled. It depends on the length of the RNA strand. The obtained cDNA was purified using magnetic purification beads. Then, 8 decreasing dilutions were performed. Each was committed in 2 replicates (n = 18) for the construction of NGS libraries targeted on the TCR beta genes. Enrichment of these genes was performed using a simplex PCR in which the sense primer is complementary to the adaptor at the 5' end of the TSO:
5'AAGACTCGGCAGCATCTCCATAGCAGGATATCAACGCAAGTA3'

It includes a 21-nucleotides adaptor at the 5' end.

The reverse primer is complementary to the *TRBC1* and *TRBC2* genes. The reverse primer also includes a 21-nucleotides adaptor at the 5'-end:
5'GCGATCGTCACTGTTCTCCATCTCTGCTTCTGATGGCTCAAAC3'.

The resulting products were purified with magnetic purification beads.

A fraction of the amplicons was engaged in a simplex PCR. The forward and reverse primers were specific to the adapters included at the 5' ends of the primers used in the enrichment PCR.

The aim of this step was to integrate the P5 and P7 adapters as well as the MIDs necessary for sequencing in the MiSeq-Illumina platform. The sequencing run was performed following the standard procedure. We used Illumina's recommended reagents in paired-end condition with a run of 2x 250 cycles. The generated reads were paired and alignment to the TCR beta genes. Then, the percentage of bases with a Phred QS < 20 was recorded for each aligned read. An average was calculated for each library. The distribution of the results obtained is represented by a boxplot in Figure 3B.

### Example 2

In this example aiming to illustrate the advantage of enriching the targeted genes in an NGS library by two nested PCRs specific to these genes versus a single specific PCR in terms of sequencing efficiency (% of on-target reads); a series of NGS libraries targeted on TCR beta genes were constructed with both approaches, respectively example 4A and 4B.

### Example 2A - Figure 4A: two nested enrichment PCRs

For example 2A - Figure 4A, the procedure for constructing libraries targeted to TCR beta genes from PBMC RNA and the sequencing conditions are identical to those described in Example 1, Figure 3A. The proportion of reads aligned to the targeted DNA sequences relative to the total raw reads (on target reads %) is calculated for each library. The distribution of results is represented by a box plot in Figure 4A.

### Comparative example 2B - Figure 4B: single enrichment PCR

In Example 2B - Figure 4B, RNA from a PBMC sample was extracted and subjected to a reverse transcription reaction using a UMI integration primer identical to that described in Example 1, Figure 3A. The obtained cDNA was purified using magnetic purification beads and then 8 decreasing dilutions were performed, and each was committed in 2 replicates (n = 18) for the construction of NGS libraries targeted on the TCR beta genes. A single enrichment PCR of the TCR beta genes is performed in this example. The same primers as those used in the 2nd enrichment PCR described in example 1A, Figure 3A.

The products of this PCR were purified using magnetic purification beads. A fraction of the resulting amplicons was engaged in a simplex PCR. The forward and reverse primers were specific for the adapters included at the 5' ends of the primers used in the enrichment PCR. The purpose of this step was to integrate the P5 and P7 adapters and the MIDs required for sequencing into the MiSeq-Illumina platform. The sequencing run was performed following the standard procedure. Illumina's recommended reagents were used in paired-end condition with a run of 2x 250 cycles. The proportion of reads aligned to TCR beta target genes to total raw reads (on target reads %) is calculated for each library. The distribution of results is represented by a boxplot in Figure 4B.

## Claims

1. A high precision method for the detection of a medical or cosmetic condition, comprising:
A. Providing a patient sample comprising a targeted DNA sequence (10); wherein the targeted DNA sequence is from 20 to 200 bases upstream and from 20 to 200 base pairs downstream from the center of a region of interest (ROI, 20), wherein the ROI (20) consists of 1 to 100, preferably from 10 to 50, and even more preferably from 20 to 50 bases; and wherein the ROI (20) is associated with the medical or cosmetic condition; and attaching a unique molecular identifier (UMI, 33, 33') to the targeted DNA sequence (10) using a UMI integration primer (30, 30'); wherein the UMI integration primer (30, 30') comprises:
▪ an amplification adapter (31, 31'),
▪ a Unique Molecular Identifier (UMI, 33, 33'),
▪ a sample identifier (32, 32') ; and
▪ a DNA sequence specific for the targeted DNA sequence (34, 34'), wherein 3'-end is neighboring the targeted DNA sequence (10).
B. Amplifying the targeted DNA sequence (10) through a first enrichment PCR thereby obtaining a first enrichment amplicon of 200 to 700 base pairs, wherein the first enrichment amplicon comprises the ROI (20);
C. Amplifying the targeted DNA sequence (10) through a second nested enrichment PCR thereby obtaining a second enrichment amplicon of 200 to 500 base pairs, wherein the second enrichment amplicon comprises the ROI (20);
D. Attaching NGS adapters (60, 61) to the second enrichment PCR amplicons; and
E. Sequencing the second enrichment amplicons through Next Generation Sequencing (NGS);
wherein the first enrichment PCR comprises amplification with:
▪ one or more first enrichment PCR primers (40), wherein the first enrichment PCR primers (40) are specific for the targeted DNA sequence (10); wherein the first enrichment PCR primers (40) are forward if the UMI integration primer (30) is downstream from the ROI (20); and wherein the first enrichment PCR primers are reverse if the UMI integration primer (30') is upstream from the ROI (20); and
▪ a primer (41) complementary to the amplification adapter (31);
wherein the second enrichment PCR comprises amplification with:
▪ one or more second enrichment PCR primers (50), wherein the second enrichment PCR primers (50) are specific for the ROI (20) or for the targeted DNA sequences (10), wherein the second enrichment PCR primers (50) are nested with respect to first enrichment PCR primers (40); and wherein the second enrichment PCR primer (50) comprises a generic adapter (51) at the 5'-end; and
▪ a primer (52) complementary to the amplification adapter (31), wherein the primer (52) comprises a generic adapter (53).

2. The method of claim 1, wherein the targeted DNA sequence (10) has a length of 20 to 200 nucleotides, preferably 20 to 100 nucleotides from the center of the ROI.

3. The method of any one of the preceding claims, wherein the ROI (20) consists of 1 to 100, preferably from 10 to 50, and even more preferably from 20 to 50 bases.

4. The method of any one of the preceding claims, wherein the DNA sequence specific for the targeted DNA sequence (34, 34') has a length of 10 to 60 nucleotides, preferably 15 to 25 nucleotides, and even more preferably 18 to 24 nucleotides.

5. The method of any one of the preceding claims, wherein the UMI has a length of 5 to 20 nucleotides, preferably 8 to 18 nucleotides, even more preferably 12 to 15, and even more preferably 14 nucleotides.

6. The method of any one of the preceding claims, wherein the UMI is an aleatory sequence, a semi-aleatory sequence or a combination thereof.

7. The method of any one of the preceding claims, wherein the random portion of the semi-aleatory sequence is from 2 to 12 bases.

8. The method of any one of the preceding claims, wherein the UMI consists of the following consecutive base order:
▪ 4 random bases,
▪ one fixed base,
▪ 4 random bases,
▪ one fixed base, and
▪ 4 random bases.

9. The method of any one of the preceding claims, wherein the region of interest is a T-cell receptor or a B-cell receptor, preferably a T-cell receptor.

10. The method of any one of the preceding claims, wherein the medical condition is cancer, preferably breast cancer or gastrointestinal cancer.

11. The method of any one of the preceding claims, wherein medical condition is recidivist cancer.

12. The method of any one of the preceding claims further comprising the determination of a treatment in function of the detected region of interest.

13. System for the high precision detection of a medical or cosmetic condition, comprising means configured to perform the following steps:
A. Providing a patient sample comprising a targeted DNA sequence (10); wherein the targeted DNA sequence is from 20 to 200 bases upstream and from 20 to 200 base pairs downstream from the center of a region of interest (ROI, 20), wherein the ROI (20) consists of 1 to 100, preferably from 10 to 50, and even more preferably from 20 to 50 bases; and wherein the ROI (20) is associated with the medical or cosmetic condition; and attaching a unique molecular identifier (UMI, 33, 33') to the targeted DNA sequence (10) using a UMI integration primer (30, 30'); wherein the UMI integration primer (30, 30') comprises:
▪ an amplification adapter (31, 31'),
▪ a Unique Molecular Identifier (UMI, 33, 33'),
▪ a sample identifier (32, 32') ; and
▪ a DNA sequence specific for the targeted DNA sequence (34, 34'), wherein 3'-end is neighboring the targeted DNA sequence (10).
B. Amplifying the targeted DNA sequence (10) through a first enrichment PCR thereby obtaining a first enrichment amplicon of 200 to 700 base pairs, wherein the first enrichment amplicon comprises the ROI (20);
C. Amplifying the targeted DNA sequence (10) through a second nested enrichment PCR thereby obtaining a second enrichment amplicon of 200 to 500 base pairs, wherein the second enrichment amplicon comprises the ROI (20);
D. Attaching NGS adapters (60, 61) to the second enrichment PCR amplicons; and
E. Sequencing the second enrichment amplicons through Next Generation Sequencing (NGS);
wherein the first enrichment PCR comprises amplification with:
▪ one or more first enrichment PCR primers (40), wherein the first enrichment PCR primers (40) are specific for the targeted DNA sequence (10); wherein the first enrichment PCR primers (40) are forward if the UMI integration primer (30) is downstream from the ROI (20); and wherein the first enrichment PCR primers are reverse if the UMI integration primer (30') is upstream from the ROI (20); and
▪ a primer (41) complementary to the amplification adapter (31);
wherein the second enrichment PCR comprises amplification with:
▪ one or more second enrichment PCR primers (50), wherein the second enrichment PCR primers (50) are specific for the ROI (20) or for the targeted DNA sequences (10), wherein the second enrichment PCR primers (50) are nested with respect to first enrichment PCR primers (40); and wherein the second enrichment PCR primer (50) comprises a generic adapter (51) at the 5'-end; and
▪ a primer (52) complementary to the amplification adapter (31), wherein the primer (52) comprises a generic adapter (53).
